# FASCICULE DE BREVET EUROPEEN

(11) **EP 2 790 659 B1**
(45) Date de publication et mention de la délivrance du brevet: **01.03.2017**
(21) Numéro de dépôt: 12801580.7
(22) Date de dépôt: 14.12.2012
(51) Int. Cl.: A61K 8/49, A61Q 19/08

(54) **COMPOSITION COSMÉTIQUE POUR STIMULER LES FONCTIONS CELLULAIRES ANTI-VIEILLISSEMENT DE LA PEAU**
KOSMETISCHE ZUSAMMENSETZUNG ZUR STIMULIERUNG DER ZELLULÄREN ALTERUNGSHEMMUNGSFUNKTIONEN DER HAUT
COSMETIC COMPOSITION FOR STIMULATING THE CELLULAR ANTI-AGING FUNCTIONS OF THE SKIN

(30) Priorité: 16.12.2011 FR 1161844
(43) Date de publication de la demande: 22.10.2014
(73) Titulaire: Syntivia, 31106 Toulouse Cedex 1 (FR); Centre National de la Recherche Scientifique, 75016 Paris (FR); Université Paul Sabatier Toulouse III, 31400 Toulouse (FR)
(72) Inventeur: BEDOS, Philippe, F-31450 Donneville (FR); BALTAS, Michel, F-31320 Castanet Tolosan (FR)
(74) Mandataire: Ipside
(86) Numéro de dépôt international: PCT/EP2012/075524
(87) Numéro de publication internationale: WO 2013/087834

(56) Documents cités:
- WO-A1-98/06695
- WO-A1-99/00079
- WO-A1-2010/110353
- JP-A- 2008 239 545
- US-B1- 6 858 642
- CHOI J Y ET AL: "Cytoprotective activities of hydroxycinnamic acid amides of serotonin against oxidative stress-induced damage in HepG2 and HaCaT cells", FITOTERAPIA, IDB HOLDING, MILAN, IT, vol. 81, no. 8, 1 décembre 2010 (2010-12-01), pages 1134-1141, XP027487785, ISSN: 0367-326X, DOI: 10.1016/J.FITOTE.2010.07.015 [extrait le 2010-07-23]
- YAMAZAKI Y ET AL: "N-[(Dihydroxyphenyl)acyl]serotonins as potent inhibitors of tyrosinase from mouse and human melanoma cells", BIOORGANIC & MEDICINAL CHEMISTRY LETTERS, PERGAMON, ELSEVIER SCIENCE, GB, vol. 19, no. 15, 1 août 2009 (2009-08-01), pages 4178-4182, XP026301661, ISSN: 0960-894X, DOI: 10.1016/J.BMCL.2009.05.115 [extrait le 2009-06-11]

## Description

La présente invention s'inscrit dans le domaine des compositions cosmétiques anti-âge. Plus particulièrement, elle concerne l'utilisation d'un composé répondant à une formule générale particulière, dans une composition cosmétique, pour prévenir et réparer les effets du vieillissement de la peau, et plus particulièrement pour stimuler les fonctions cellulaires anti-vieillissement de la peau.

La peau est la première barrière protégeant le corps contre les agressions extérieures. Le phénomène de vieillissement cutané, qu'il soit naturel ou induit par ces agressions extérieures, affaiblit cette fonction barrière. Les symptômes de ce vieillissement sont notamment l'apparition de rides, la sécheresse, la rugosité, l'amincissement et la perte d'élasticité de la peau. Le traitement de ces symptômes est devenu un enjeu majeur pour l'industrie cosmétique.

Cependant, si la connaissance de la physiologie de la peau a permis de proposer des solutions cosmétiques à différents dysfonctionnements induits par les agressions extérieures, il n'a été proposé à l'heure actuelle par l'art antérieur aucune solution satisfaisante pour prévenir et réparer les effets du vieillissement de la peau.

Parmi les principaux actifs cosmétiques proposés par l'art antérieur pour améliorer l'aspect de la peau, on peut citer par exemple les composés d'origine végétale tels que les polyphénols, dont l'activité antioxydante a été mise à profit pour prévenir le stress oxydatif au niveau de la peau, par piégeage des radicaux libres résultant d'agressions extérieures, par exemple d'une exposition de la peau aux rayons ultra-violets (UV). L'acide caféique est un exemple d'un tel polyphénol mis en oeuvre dans des compositions cosmétiques dites anti-âge proposées par l'art antérieur. Les compositions cosmétiques à base de tels composés antioxydants permettent, dans une certaine mesure, de ralentir l'apparition des symptômes du vieillissement. Leur efficacité est cependant limitée, et elles ne permettent en outre pas de réparer les effets du vieillissement.

La présente invention vise à remédier aux inconvénients des compositions cosmétiques dites anti-âge proposées par l'art antérieur, notamment à ceux exposés ci-avant, en proposant une telle composition qui permette de lutter efficacement contre les effets du vieillissement cutané, en en prévenant, et même en en réparant, les symptômes.

Il a maintenant été découvert par les présents inventeurs qu'une certaine classe de composés, répondant à une formule générale particulière, permettaient, mis en oeuvre en tant que principes actifs dans des compositions cosmétiques, d'atteindre un tel résultat.

La présente invention concerne ainsi l'utilisation d'un composé de formule générale (I) : 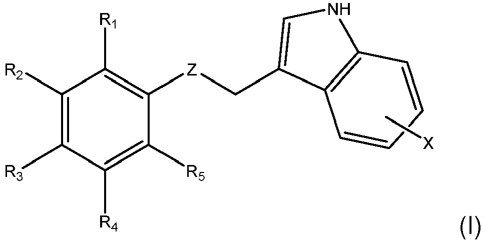 dans laquelle :
R₁, R₂, R₃, R₄, R₅, identiques ou différents, représentent chacun un atome d'hydrogène, un atome d'halogène, tel qu'un atome de fluor, de chlore, de brome ou d'iode, un groupement hydroxyle ou un radical -OR' où R' représente un radical hydrocarboné linéaire ou ramifié, saturé ou insaturé, en C1-C16, au moins un groupement parmi R₁, R₂, R₃, R₄ et R₅ ne représentant pas un atome d'hydrogène ou un atome d'halogène,
X représente un atome d'halogène, un groupement hydroxyle, un groupement nitro, un radical hydrocarboné linéaire ou ramifié, saturé ou insaturé, éventuellement substitué, en C1-C14, ou un radical -OR" où R" représente un radical hydrocarboné linéaire ou ramifié, saturé ou insaturé, éventuellement substitué, en C1-C14,
Z représente une liaison covalente ou un bras espaceur,
ou un de ses sels,
dans une composition cosmétique pour stimuler les fonctions cellulaires anti-vieillissement de la peau.

On entend, par « stimuler les fonctions cellulaires anti-vieillissement de la peau », le fait que les composés selon l'invention agissent sur les cellules de la peau, pour en stimuler des fonctions naturelles qui sont impliquées dans les mécanismes de lutte contre les symptômes du vieillissement de la peau et de réparation de ces symptômes. Plus particulièrement, il a été découvert par les présents inventeurs que les composés selon l'invention infèrent sur l'expression de gènes impliqués dans ces mécanismes, dans un sens favorisant la protection contre les effets du vieillissement cutané, et même la réparation des symptômes qui y sont liés. Ainsi, les composés selon l'invention, appliqués sur la peau par voie topique, induisent la surexpression de certains gènes des cellules de la peau codant des protéines à effet anti-vieillissement cutané, et l'inhibition de l'expression d'autres gènes codant des protéines provoquant des symptômes du vieillissement. Ces composés, mis au contact des cellules de la peau, ont notamment pour effet d'induire une augmentation de la production, par ces cellules, de protéines à effet anti-vieillissement cutané, telles que le collagène. L'action des composés selon l'invention s'exprime notamment par un renforcement de la fonction barrière de la peau, l'induction de la néosynthèse de protéines essentielles de la matrice extracellulaire cutanée, et l'activation de protéines régénératrices ou détoxifiantes au niveau de la peau.

A titre de gènes dont la surexpression est induite par un composé selon l'invention, on peut citer par exemple :
- le gène codant la sirtuine 5, enzyme mitochondriale qui augmente la survie cellulaire en retardant le phénomène d'apoptose, plus particulièrement par désacétylation du cytochrome C, protéine ayant un rôle central dans l'induction de l'apoptose (Schlicker et al., 2008). La surexpression de cette enzyme favorise la survie cellulaire.
- le gène codant la protéine Klotho, hormone présentant la capacité d'accroître la survie cellulaire, par son implication dans la lutte contre le stress oxydatif, la régulation de la croissance cellulaire et celle des canaux ioniques (Kuro et al., 2009).
- des gènes impliqués dans la différenciation et l'adhésion des kératinocytes, qui entrent dans la constitution de l'épiderme, couche de la peau en assurant principalement la fonction barrière. Avec l'âge, cette barrière s'affaiblit, entraînant une fragilisation et une déshydratation de l'épiderme. La cohésion de l'épiderme est assurée par plusieurs structures d'adhésion intercellulaires. Au cours du temps, cette cohésion cellulaire se relâche, affectant la fonction barrière et l'étanchéité de la peau. Une augmentation de l'expression de protéines impliquées dans la différenciation et l'adhésion des kératinocytes favorise la formation et l'étanchéité de la barrière cutanée, ce qui permet, d'une part, de maintenir l'hydratation de la peau, et, d'autre part, de protéger la peau des agressions extérieures et de contrer son vieillissement. De tels gènes sont notamment les gènes codant l'Epiplakine 1 (EPPK1), protéine associée aux filaments intermédiaires (FI) de Kératine et de Vimentine, qui participe au maintien du cytosquelette au cours de la différenciation des kératinocytes (Jang et al., 2005) ; l'Envoplakine (EVPL), qui participe à la formation des desmosomes, au sein desquels elle permet la liaison des filaments intermédiaire (FI) de kératine (Karashima et al., 2002) ; la Transglutaminase 1 (TGM1), enzyme capable de catalyser la liaison entre les précurseurs de l'enveloppe cornée et la membrane cellulaire, jouant un rôle clé dans la différenciation, et favorisant la formation de la barrière cutanée et l'hydratation de la peau (Eckert et al., 2005) ; la Serine peptidase inhibitor, Kazal type 5 (SPINK5), protéine inhibitrice de la desquamation, exprimée au sein des cornéocytes et participant au maintien de la couche cornée et de l'hydratation de la peau (Chavanas et al, 2000) ; l'aquaporine 3 (AQP3), molécule intermembranaire spécifique de l'épiderme qui forme, au niveau de la paroi cellulaire, des tunnels spécialisés dans le passage de l'eau, permettant de conserver un équilibre hydrique dans la peau et ceci même dans des conditions défavorables, l'altération de son métabolisme, notamment due à l'âge, provoquant une perturbation du gradient hydrique se traduisant par une mauvaise circulation de l'eau (Hara-Chikuma et al., 2005) ; les Cornulines (CRNN), marqueurs de la différenciation terminale, également connues sous le nom de Sepp53 (pour l'anglais squamous epithelial heat shock protein 53) (Contzler et al., 2005) ; la Kératine 1 (KRT1), protéine participant à la formation de l'enveloppe cornée et à la fonction barrière (Senshu et al., 1996).
- des gènes codant des protéines de la matrice extracellulaire (MEC). La matrice extracellulaire est constituée de nombreuses protéines qui rendent la peau ferme et tonique tout en restant élastique. Les protéines les plus connues sont les collagènes pour le pouvoir gainant, l'élastine aux propriétés élastiques et l'acide hyaluronique pour son effet structurant. Avec le temps, ces protéines structurales ne se renouvellent plus et finissent par être dégradées, ce qui entraine une perte d'élasticité, une déstructuration de la peau et l'apparition de rides. En particulier, les composés selon l'invention sont capables d'augmenter l'expression des gènes de la MEC et de favoriser la néosynthèse de ces protéines structurelles. De tels gènes sont notamment les gènes codant les collagènes 1 3 et 5 (COL1A1, COL3A1, COL5A1), protéines essentielles de la structure de la MEC, qui ont également pour fonction de conférer à la peau une résistance mécanique à l'étirement (Tzaphlidou, 2004, Kuo et al., 1997) ; l'élastine (ELN), protéine élastique de la MEC assurant l'élasticité de la peau (Uitto et al., 1979) ; la laminine alpha 5 (LAMA5), qui favorise l'adhésion cellulaire au niveau de la jonction dermo-épidermique et le maintien de l'intégrité de la peau (Schneider et al., 2006) ; l'hyaluronan synthase 3 (HAS3), enzyme responsable de la synthèse d'acide hyaluronique, constituant essentiel de la MEC responsable entre autre de l'hydratation de la peau (Manuskiatti et al., 1996) ; la secreted protein acidic cystein-rich, ou osteonectine (SPARC), protéine exprimée dans les tissus à renouvellement permanent, tels que la peau et au niveau des sites de traumatisme ou remaniement, qui se fixe sur divers constituants de la matrice extracellulaire (collagènes fibrillaires, collagène IV, thrombospondine-1, vitronectine, etc.) et participe à son organisation (Bradshaw et al., 2002).

A titre de gènes dont l'expression serait inhibée par un composé selon l'invention, on peut citer par exemple le gène codant la Matrice-Métalloprotéase de type 9 (MMP-9), enzyme connue pour dégrader les composants de la Matrice Extra-Cellulaire (MEC) assurant la structure du derme, sous la membrane basale de l'épiderme, notamment le collagène (Seltzer et al., 1989), et pour participer au vieillissement de la peau. Le stress UV augmente l'expression de MMP-9. L'inhibition de l'expression du gène responsable de la production de la MMP-9, médiée par les composés conformes à l'invention, provoque un ralentissement de la dégradation des protéines de structure du derme, préservant plus longtemps l'intégrité du derme dans le temps.

Suivant des modes de mise en oeuvre particuliers, l'invention répond en outre aux caractéristiques suivantes, mises en oeuvre séparément ou en chacune de leurs combinaisons techniquement opérantes.

Dans des modes de mise en oeuvre particuliers de l'invention, Z représente un bras espaceur portant une fonction amide. Préférentiellement, le composé répond alors à la formule (II) : 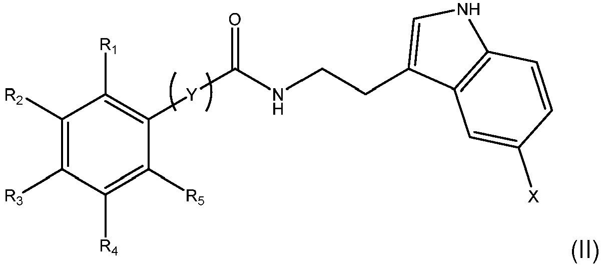 dans laquelle Y représente une liaison covalente ou un radical hydrocarboné linéaire ou ramifié, saturé ou insaturé, en C1-C16.

Préférentiellement, dans cette formule (II), au moins un parmi R₃, R₄ et R₅ représente un groupement hydroxyle, X représente un groupement hydroxyle ou un groupement méthoxy, et Y représente une liaison covalente ou un radical alcényle en C2-C4.

Ainsi, dans des modes de mise en oeuvre particuliers de l'invention, un composé conforme à la présente invention répond à la formule générale (III) ci-après : 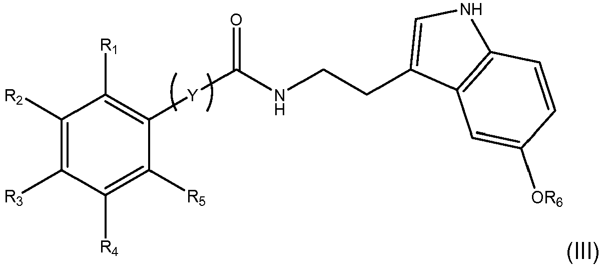 dans laquelle :
R₁, R₂, R₃, R₄, R₅, identiques ou différents, représentent chacun un atome d'hydrogène, un atome d'halogène, tel qu'un atome de fluor, de chlore, de brome ou d'iode, un groupement hydroxyle ou un radical -OR' où R' représente un radical hydrocarboné linéaire ou ramifié, saturé ou insaturé, en C1-C16, au moins un groupement parmi R₃, R₄ et R₅ représentant un groupement hydroxyle,
R₆ représente un atome d'hydrogène ou un groupement méthyle,
et Y représente une liaison covalente ou un radical alcényle en C2-C4, de préférence en C2.

Préférentiellement, dans la formule (III) ci-dessus, au moins R₁ et R₂ représentent chacun un atome d'hydrogène.

Des composés particulièrement avantageux dans le cadre d'une utilisation cosmétique, en termes d'efficacité de la stimulation des fonctions cellulaires anti-vieillissement de la peau, répondent respectivement à la formule (IIa) : 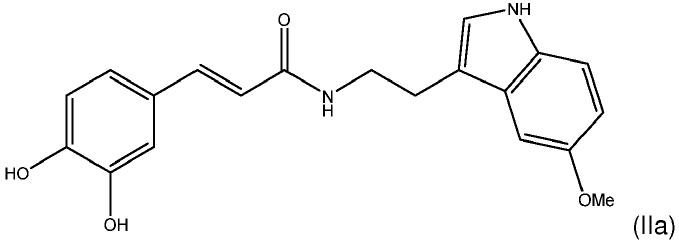 et à la formule (IIb) :

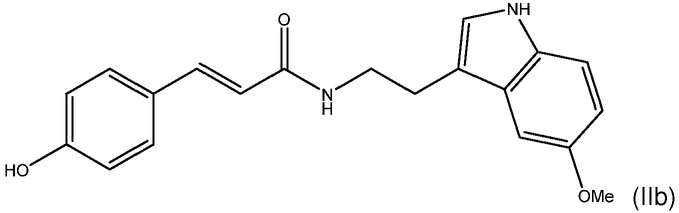

Les composés conformes à l'invention peuvent être obtenus selon toute méthode connue de l'homme du métier, par exemple par synthèse chimique, synthèse enzymatique, par fermentation d'une souche de bactéries, modifiées ou non par voie génétique, etc.

Dans des modes de mise en oeuvre particuliers de l'invention, particulièrement avantageux en termes d'efficacité de prévention et de réparation des effets du vieillissement cutané, le composé est présent dans la composition cosmétique à une concentration comprise entre 0,0000001 et 10 % en poids du poids total de la composition, de préférence à une concentration comprise entre 0,00001 et 1 % en poids du poids total de la composition.

Plusieurs des composés conformes à l'invention peuvent être utilisés simultanément, formulés dans la même composition cosmétique.

La composition cosmétique à base d'au moins un composé conforme à l'invention est préférentiellement destinée à un usage topique. Le composé y est présent dans un véhicule cosmétiquement acceptable classique en lui-même. Par l'expression « véhicule cosmétiquement acceptable », on entend que le véhicule est adapté à une utilisation par mise en contact avec des cellules humaines et animales, en particulier les cellules de la peau. De préférence, ce véhicule présente une odeur, une couleur et un toucher agréables, il ne génère pas d'inconforts inacceptables susceptibles de détourner un utilisateur de la composition.

La composition peut se présenter sous toute forme classique en elle-même, notamment, mais non limitativement, sous forme de crème, de pommade, de lait, d'huile, d'onguent, de lotion, de poudre, de solution, de gel, de suspension, de savon, de tampon imbibé, ou encore de shampooing.

Elle peut en outre comporter tout additif classique en lui-même dans le domaine de la cosmétique, tel qu'un diluant, un agent conservateur, stabilisateur, émulsifiant, adjuvant, transporteur, etc.

L'effet cosmétique recherché peut être renforcé par la mise en oeuvre dans la composition de tout autre ingrédient actif supplémentaire, présentant un effet bénéfique pour la peau, cet effet pouvant ou pas s'exercer de façon synergique avec celui du composé conforme à l'invention. Un tel ingrédient actif supplémentaire peut notamment présenter une activité de réduction des rides, d'augmentation de l'hydratation de la peau, de sa fermeté, de renforcement de sa fonction barrière, d'épaississement de la peau, etc. En tant qu'ingrédient actif pouvant être mis en oeuvre en association ou en combinaison avec un composé conforme à l'invention, on peut citer, à titre d'exemple, les agents anti-âges, les agents anti-rides, les agents desquamants, les agents hydratants, les agents dépigmentants, les agents propigmentants, les agents anti-glycation, les inhibiteurs de NO-synthase, les agents stimulant la synthèse de macromolécules dermiques ou épidermiques et/ou empêchant leur dégradation, les agents stimulant la prolifération des fibroblastes et/ou des kératinocytes ou stimulant la différenciation des kératinocytes, les agents myorelaxants, les agents dermo-décontractants, les agents tenseurs, les agents anti-pollution et/ou anti-radicalaires, les agents anti-irritants, les agents agissant sur la microcirculation, les agents agissant sur le métabolisme énergétique des cellules, les agents anti-UV et leurs mélanges, une telle liste n'étant nullement limitative.

De tels agents peuvent être choisis parmi les caroténoïdes (par exemple beta-carotène, lycopène, astaxanthine, phytoènes), les rétinoïdes (par exemple rétinol, vitamine A, acide rétinoïque cis ou trans, esters de rétinol), les flavanones, les flavonols, les isoflavones (par exemple genisteine, daidzeine, rutine, etc.), les coumarines, les lignanes, les vitamines (par exemple A, B, C, E, F, K, H), les stilbenoïdes, les sapogénines, les acides triterpéniques pentacycliques, les β-hydroxyacides, les hydroxyphénols et leurs dérivés éthers, ester ou hétérosides, les acides phénoliques, les monomères précurseurs des tanins, les aminosucres, les aminoacides (par exemple arginine, lysine, tyrosine, cystéine, taurine, etc.), les peptides (par exemple carnosine, enképhalines, les peptides commerciaux proposés pour leur effet anti-âge tel que la Pal-KTTKS (Matrixyl® de la société Sederma), Ac-Hexapeptide 3 (Argireline de la société Lipotec), Pal-GQPR (Rigin® de la société Sederma), Dermican, Ac-tetrapeptide 9 (Société BASF Beauty Solutions), Syn-ake, tripeptide (Société DMS/Pentapharm), et leurs mélanges, etc.

Un autre aspect de l'invention concerne une composition cosmétique pour stimuler les fonctions cellulaires anti-vieillissement de la peau, comportant un composé de formule générale (I) : 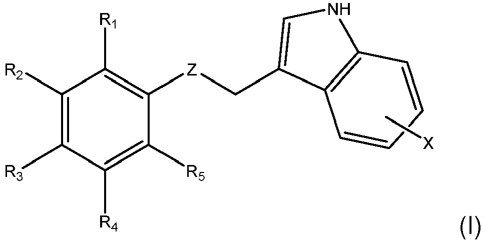 dans laquelle :
R₁, R₂, R₃, R₄, R₅, identiques ou différents, représentent chacun un atome d'hydrogène, un atome d'halogène, tel qu'un atome de fluor, de chlore, de brome ou d'iode, un groupement hydroxyle ou un radical -OR' où R' représente un radical hydrocarboné linéaire ou ramifié, saturé ou insaturé, en C1-C16, au moins un groupement parmi R₁, R₂, R₃, R₄ et R₅ ne représentant pas un atome d'hydrogène ou un atome d'halogène,
X représente un atome d'halogène, un groupement hydroxyle, un groupement nitro, un radical hydrocarboné linéaire ou ramifié, saturé ou insaturé, éventuellement substitué, en C1-C14, ou un radical -OR" où R" représente un radical hydrocarboné linéaire ou ramifié, saturé ou insaturé, éventuellement substitué, en C1-C14,
Z représente une liaison covalente ou un bras espaceur,
ou un de ses sels,
dans un véhicule cosmétiquement acceptable.

Cette composition peut répondre à l'une ou plusieurs des caractéristiques énoncées ci-avant. En particulier, le composé peut répondre à l'une des formules (II) ou (III) ci-dessus.

Un procédé d'utilisation de cette composition cosmétique prévoit l'application par voie topique, sur les parties du corps concernées, par exemple sur le visage, d'une quantité déterminée de composition, à raison d'une ou deux fois par jour, par exemple le matin et le soir, pendant une période comprise entre 2 semaines et 2 mois ou plus.

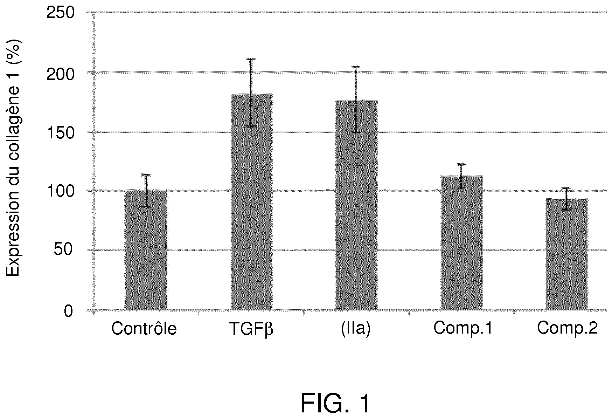

Les caractéristiques et avantages de l'invention apparaîtront plus clairement à la lumière des exemples de mise en oeuvre ci-après, fournis à simple titre illustratif et nullement limitatifs de l'invention, avec l'appui de la figure 1 qui est un graphe en barres montrant l'expression du collagène 1 par des cellules de fibroblastes dermiques humains normaux, après incubation *in vitro* en absence (contrôle) ou en présence des composés : TGFβ (témoin positif), composé conforme à l'invention (IIa), composés comparatifs Comp.1 et Comp.2, le contrôle étant rapporté à 100 %.

### EXEMPLES 1-8 - Synthèse chimique de composés conformes à des modes de mise en oeuvre de l'invention et de composés comparatifs

### Protocole de synthèse

Pour tous les Exemples ci-après, le protocole de synthèse est le suivant.

Dans un ballon à l'intérieur duquel on a placé un barreau aimanté, sont pesés successivement A mg de composant A, puis D mg de composant D. Le ballon est mis sous atmosphère inerte.

Parallèlement, dans un second ballon à l'intérieur duquel on a placé un barreau aimanté, sont pesés B mg de composant B, puis C mg de composant C. Le ballon est également mis sous atmosphère inerte.

On ajoute ensuite F mL de diméthylformamide (DMF) anhydre dans le premier ballon. Dans le second ballon, on ajoute E mL de dichlorométhane (DCM) distillé, puis G µL de triéthylamine (Et₃N). Les deux ballons sont alors mis sous agitation magnétique en bain d'huile à 50 °C pendant 1 h, puis le contenu du second ballon est additionné rapidement à la seringue dans le premier ballon. Le mélange est alors placé à reflux en bain à 50 °C.

La réaction est suivie sur Chromatographie sur Couche Mince (CCM) (éluant : 7,5AE/1MeOH/1,5chloroforme + 1,5 % d'acide trifluoroacétique (TFA) ; révélation à 254 nm puis à la ninhydrine).

Après 3 h de réaction, on laisse la solution revenir à température ambiante.

On ajoute ensuite 50 mL d'acétate d'éthyle (AE) dans le brut réactionnel. On réalise trois opérations d'extraction successives avec 20 mL de solution aqueuse d'acide chlorhydrique (HCl) à 1 M, puis des lavages avec 20 mL de solution de carbonate de sodium (NaHCO₃) saturée, puis avec 20 mL d'H₂O. On récupère la phase organique, que l'on concentre sous pression réduite. On ajoute 10 mL de méthanol (MeOH) et on réalise une nouvelle concentration sous pression réduite.

On réalise une purification par chromatographie sur colonne de silice 70-200 µm, avec pour éluants : Eluant n°1 : AE ; Eluant n°2 : (7,5AE/1MeOH/1,5chloroforme) + 1,5%TFA ; Eluant n°3 : (9AE/1MeOH) + 3% TFA.

On récupère les fractions d'intérêt que l'on combine et auxquelles on ajoute 20 mL d'AE. On réalise quatre extractions avec 20 mL de solution de NaHCO₃ saturée, puis des lavages avec 2 x 20 mL d'H₂O. On récupère la phase organique, que l'on concentre sous pression réduite. On ajoute 10 mL d'éthanol (EtOH) et on réalise une nouvelle concentration sous pression réduite, pour obtenir le composé désiré.

### Exemple 1 - 2-Propenamide, 3-(3,4-dihydroxyphenyl)-N-[2-(5-methoxy-1 H-indol-3-yl)ethyl]-, (2E)-

Ce composé conforme à l'invention, de formule (IIa) : 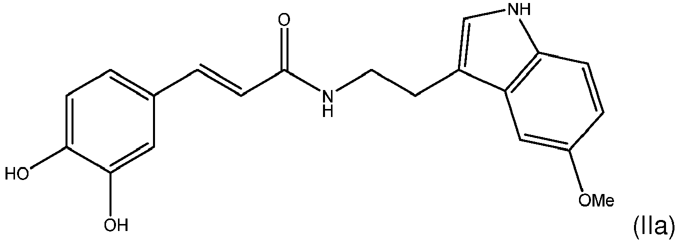 dans laquelle R₁, R₂, R₅ représentent chacun un atome d'hydrogène, R₃ et R₄ représentent chacun un groupement hydroxyle, X représente un groupement méthoxy et Y représente un radical éthylényle, est préparé comme indiqué dans le protocole ci-dessus, avec les composants dans les quantités suivantes :
Composant A : acide caféique / 500 mg
Composant B : méthoxytryptamine (97 %) / 847 mg
Composant C : 1-éthyl-3-(3-diméthylaminopropyl)carbodiimide (EDC.HCl) (98 %) / 846 mg
Composant D : hydroxybenzotriazole (HOBt) (98 %) / 464 mg
DCM distillé : Volume E = 12,4 ml
DMF anhydre : Volume F = 6,2 ml
Et₃N : Volume G = 970 µl

On récupère finalement 586 mg d'un solide marron (M = 352,39 g.mol⁻¹ ; tr(LCMS) = 5,2 min ; p = 60 % ; pureté LC > 95 %).

La méthode d'analyse chromatographique (LCMS) est la suivante : Colonne C18 XBridge Waters 150x4,6 mm 5 µm; éluants : eau + 0,1% acide formique (HCOOH) / acétonitrile + 0,1 % HCOOH ; débit 1 ml/min ; début du gradient : 95-5, au bout de 9 min : 5-95, puis gradient isocratique pendant 30 secondes : 5-95, et retour à 95-5 en 1 min. Détection par détecteur UV.

Analyse RMN du proton (¹H) : 3.605 (1, 3H), 6.528 (5, 1H, d, J=15.688), 7.502 (6, 1 H, d, J=15.688), 7.657 (7, 1 H, dd, J=8.447, J=1.944), 6.763 (8, 1 H, dd, J=8.447, J=1.177), 6.754 (9, 1 H, dd, J=8.451, J=1.651), 7.020 (10, 1 H, ddd, J=8.451, J=5.495, J=5.376), 7.286 (11, 1H, dd, J=5.495, J=4.570), 7.253 (12, 1H, dd, J=1.944, J=1.177), 7.114 (13, 1H, ddd, J=5.376, J=4.570, J=1.651), 3.478 (14, 2H, t, J=6.356), 2.913 (15, 2H, t, J=6.356)

### Exemple 2

On prépare le composé conforme à l'invention de formule (IIc) : 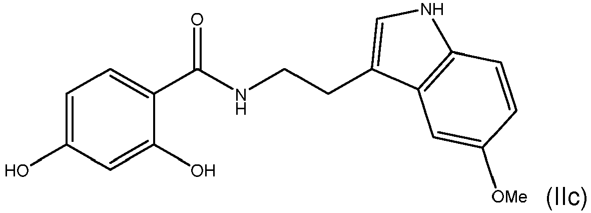 dans laquelle R₁, R₂, R₄ représentent chacun un atome d'hydrogène, R₃ et R₅ représentent chacun un groupement hydroxyle, X représente un groupement méthoxy et Y représente une liaison covalente, comme indiqué dans le protocole général ci-dessus, avec les composants dans les quantités suivantes :
Composant A : acide 2,4-dihydroxybenzoïque / 48 mg
Composant B : méthoxytryptamine (97 %) / 80 mg
Composant C : EDC.HCl (98 %) / 92 mg
Composant D : HOBt (98 %) / 58 mg
DCM distillé : Volume E = 2,5 ml
DMF anhydre : Volume F = 1,5 ml
Et₃N : Volume G = 0,1 ml

On récupère 65,8 mg d'un solide (M = 326,4 g.mol⁻¹ ; tr(LCMS) = 7,5 min ; p = 70 % ; pureté LC > 95 %)

Analyse RMN du proton : 3.605 (1, 3H), 6.942 (5, 1 H, dd, J=8.099, J=2.467), 7.613 (6, 1 H, dd, J=8.099, J=0.494), 6.815 (7, 1 H, dd, J=8.497, J=1.622), 6.942 (8, 1 H, ddd, J=8.497, J=2.756, J=1.268), 7.371 (9, 1 H, dd, J=5.280, J=2.756), 6.515 (10, 1 H, dd, J=2.467, J=0.494), 7.114 (11, 1 H, ddd, J=5.280, J=1.622, J=1.268), 3.507 (12, 2H, t, J=6.369), 2.915 (13, 2H, t, J=6.369)

### Exemple 3

On prépare le composé conforme à l'invention de formule (IId) : 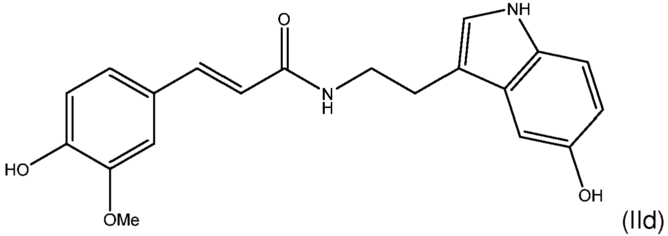 dans laquelle R₁, R₂, R₅ représentent chacun un atome d'hydrogène, R₃ représente un groupement hydroxyle et R₅ représente un groupement méthoxy, X représente un groupement hydroxyle et Y représente un radical éthylènyle, comme indiqué dans le protocole général ci-dessus, avec les composants dans les quantités suivantes :
Composant A : acide férulique / 33 mg
Composant B : sérotonine, HCl (97 %) / 51 mg
Composant C : EDC.HCl (98 %) / 54 mg
Composant D : HOBt (98 %) / 28 mg
DCM distillé : Volume E = 1,5 ml
DMF anhydre : Volume F = 0,5 ml
Et₃N : Volume G = 0,08 ml

On récupère finalement 40 mg d'un solide marron (M = 352,14 g.mol⁻¹ ; tr(LCMS) = 6,6 min ; p = 68 % ; pureté LC > 95 %)

RMN du proton : 3.784 (1, 3H), 6.528 (5, 1 H, d, J=15.683), 7.514 (6, 1 H, d, J=15.683), 7.588 (7, 1 H, dd, J=8.449, J=1.944), 6.776 (8, 1 H, dd, J=8.449, J=0.644), 6.780 (9, 1 H, dd, J=8.440, J=1.935), 7.029 (10, 1 H, ddd, J=8.440, J=5.498, J=0.631), 7.241 (11, 1 H, dd, J=3.967, J=0.631), 7.229 (12, 1 H, dd, J=1.944, J=0.644), 7.129 (13, 1 H, ddd, J=5.498, J=3.967, J=1.935), 3.497 (14, 2H, t, J=6.274), 2.922 (15, 2H, t, J=6.274)

### Exemple 4

On prépare le composé conforme à l'invention de formule (IIe) : 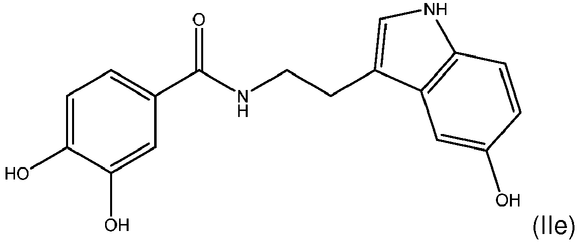 dans laquelle R₁, R₂, R₅ représentent chacun un atome d'hydrogène, R₃ et R₄ représentent chacun un groupement hydroxyle, X représente un groupement hydroxyle et Y représente une liaison covalente, comme indiqué dans le protocole général ci-dessus, avec les composants dans les quantités suivantes :
Composant A : acide 2,4-dihydroxybenzoïque / 50 mg
Composant B : sérotonine, HCl (97 %) / 86 mg
Composant C : EDC.HCl (98 %) / 94 mg
Composant D : HOBt (98 %) / 54 mg
DCM distillé : Volume E = 2,5 ml
DMF anhydre : Volume F = 1,5 ml
Et₃N : Volume G = 0,16 ml

On récupère finalement 42 mg d'un solide (M = 312,13 g.mol⁻¹ ; tr(LCMS) = 5,6 min ; p = 42 % ; pureté LC > 95 %)

RMN du proton : 6.965 (5, 1 H, dd, J=8.393, J=1.950), 7.323 (6, 1 H, dd, J=8.393, J=1.631), 6.829 (7, 1 H, dd, J=8.461, J=1.284), 6.897 (8, 1 H, ddd, J=8.461, J=2.432, J=1.890), 7.388 (9, 1H, dd, J=5.485, J=2.432), 7.455 (10, 1 H, dd, J=1.950, J=1.631), 7.129 (11, 1 H, ddd, J=5.485, J=1.890, J=1.284), 3.519 (12, 2H, t, J=6.280), 2.924 (13, 2H, t, J=6.280)

### Exemple 5

On prépare le composé conforme à l'invention de formule (IIf) : 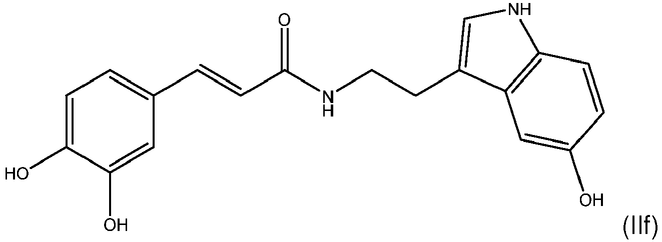 dans laquelle R₁, R₂, R₅ représentent chacun un atome d'hydrogène, R₃ et R₄ représentent chacun un groupement hydroxyle, X représente un groupement hydroxyle et Y représente un radical éthylényle, comme indiqué dans le protocole général ci-dessus, avec les composants dans les quantités suivantes :
Composant A : acide 2,4-dihydroxycinnamique / 60 mg
Composant B : sérotonine, HCl (97 %) / 86 mg
Composant C : EDC.HCl (98 %) / 92 mg
Composant D : HOBt (98 %) / 58 mg
DCM distillé : Volume E = 2,5 ml
DMF anhydre : Volume F = 1,5 ml
Et₃N : Volume G = 0,106 ml

On récupère finalement 70 mg d'un solide (M = 338,37 g.mol⁻¹ ; tr(LCMS) = 5,9 min ; p = 65 % ; pureté LC > 95 %)

RMN du proton: 6.528 (5, 1H, d, J=15.688), 7.503 (6, 1H, d, J=15.688), 7.657 (7, 1 H, dd, J=8.447, J=1.944), 6.780 (8, 1 H, dd, J=8.440, J=1.935), 6.763 (9, 1H, dd, J=8.447, J=1.177), 7.029 (10, 1H, ddd, J=8.440, J=5.498, J=0.631), 7.240 (11, 1H, dd, J=3.967, J=0.631), 7.253 (12, 1H, dd, J=1.944, J=1.177), 7.129 (13, 1 H, ddd, J=5.498, J=3.967, J=1.935), 3.497 (14, 2H, t, J=6.274), 2.922 (15, 2H, t, J=6.274)

### Exemple 6

On prépare le composé conforme à l'invention de formule (IIb) : 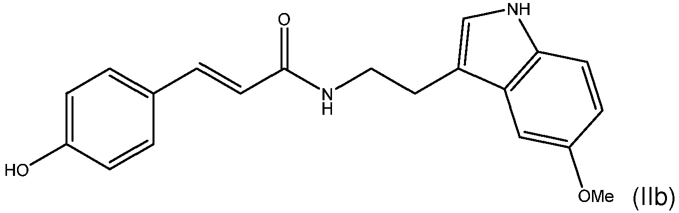 dans laquelle R₁, R₂, R₄ et R₅ représentent chacun un atome d'hydrogène, R₃ représente un groupement hydroxyle, X représente un groupement méthoxy et Y représente un radical éthylényle, comme indiqué dans le protocole général ci-dessus, avec les composants dans les quantités suivantes :
Composant A : acide coumarique / 27 mg
Composant B : méthoxytryptamine / 47 mg
Composant C : EDC.HCl (98 %) / 47 mg
Composant D : HOBt (98 %) / 27 mg
DCM distillé : Volume E = 1,5 ml
DMF anhydre : Volume F = 0,5 ml
Et₃N : Volume G = 0,08 ml

On récupère finalement 30 mg d'un solide (M = 336,14 g.mol⁻¹ ; tr(LCMS) = 7,4 min ; p = 58 % ; pureté LC > 95 %)

RMN du proton : 3.605 (1, 3H), 6.521 (4, 1 H, d, J=15.574), 7.488 (5, 1 H, d, J=15.574), 6.888 (6, 1 H, ddd, J=8.017, J=0.445, J=0.000), 6.888 (7, 1 H, ddd, J=8.017, J=0.448, J=0.000), 7.527 (8, 1 H, ddd, J=8.017, J=0.448, J=0.000), 7.527 (9, 1H, ddd, J=8.017, J=0.445, J=0.000), 6.754 (10, 1H, dd, J=8.451, J=1.651), 7.020 (11, 1 H, ddd, J=8.451, J=5.495, J=5.376), 7.286 (12, 1 H, dd, J=5.495, J=4.570), 7.114 (13, 1 H, ddd, J=5.376, J=4.570, J=1.651), 3.477 (14, 2H, t, J=6.358), 2.913 (15, 2H, t, J=6.358).

### Exemple 7 - Composé comparatif Comp.1

On prépare le composé comparatif Comp.1, 2-propenamide, 3-(3-hydroxy-4-léthoxyphényl)-N-[2-(1 H-indol-3-yl)éthyl]-, (2E)-, de formule (IV) : 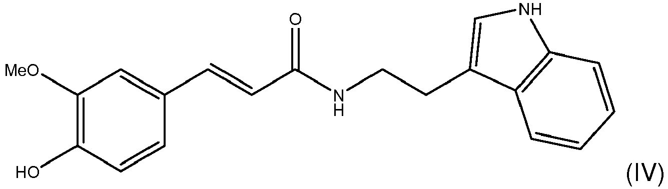 dans laquelle le noyau indole ne comporte aucun substituant, comme indiqué dans le protocole général ci-dessus, avec les composants dans les quantités suivantes :
Composant A : acide férulique / 194 mg
Composant B : tryptamine (97 %) / 264 mg
Composant C : EDC.HCl (98 %) / 287 mg
Composant D : HOBt (98 %) / 162 mg
DCM distillé : Volume E = 3 ml
DMF anhydre : Volume F = 1 ml
Et₃N : Volume G = 0,35 ml

On récupère 60 mg d'un solide marron beige (M = 336,38 g.mol⁻¹ ; tr(LCMS) = 9,5 min ; p = 18 % ; pureté LC > 92 %)

Analyse RMN du proton : 2.86 (13, 2H, t, J=7.1), 3.49 (12, 2H, t, J=7.1), 3.83 (24, 3H, s), 5.35 (7, 1 H, s), 6.46 (27, 1 H, d, J=15.1), 6.79 (4, 1 H, dd, J=7.5, J=1.5), 6.99 (5, 1 H, d, J=7.5), 7.11 (20 et 21, 2H, ddd, J=7.5, J=7.5, J=1.5), 7.16 (2, 1 H, d, J=1.5), 7.32 (22, 1 H, dd, J=7.5, J=1.5), 7.37 (26, 1 H, d, J=15.1), 7.47 (15, 1 H, s), 7.60 (19, 1 H, dd, J=7.5, J=1.5), 8.03 (11, 1 H, s), 10.10 (16, 1 H, s)

### Exemple 8 - Composé comparatif Comp.2

On prépare le composé comparatif Comp.2, 4-hydroxy-N-[2-(1H-indol-3-yl)éthyl]benzamide, de formule (V) : 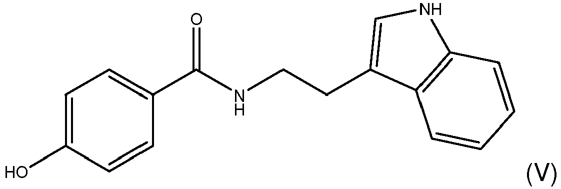 dans laquelle le noyau indole ne comporte aucun substituant, comme indiqué dans le protocole général ci-dessus, avec les composants dans les quantités suivantes :
Composant A : acide p-hydroxybenzoïque / 138 mg
Composant B : tryptamine (97 %) / 264 mg
Composant C : EDC.HCl (98 %) / 287 mg
Composant D : HOBt (98 %) / 162 mg
DCM distillé : Volume E = 3 ml
DMF anhydre : Volume F = 1 ml
Et₃N : Volume G = 0,35 ml

On récupère 200 mg d'un solide marron beige (M = 280,32 g.mol⁻¹ ; tr(LCMS) = 8,9 min ; ρ = 68 % ; pureté LC > 97 %)

Analyse RMN du proton : 2.90 (11, 2H, t, J=7.1), 3.55 (10, 2H, t, J=7.1), 5.35 (7, 1 H, s), 6.88 (1 et 5, 1 H, dd, J=7.5, J=1.5), 7.11 (18 et 19, 2H, ddd, J=7.5, J=7.5, J=1.5), 7.32 (20, 1 H, dd, J=7.5, J=1.5), 7.47 (13, 1 H, s), 7.60 (17, 1 H, dd, J=7.5, J=1.5), 7.86 (2 et 4, 2H, dd, J=7.5, J=1.5), 8.03 (9, 1 H, s), 10.10 (14, 1H, s).

EXEMPLES 9-11 - Compositions cosmétiques à base d'un composé selon des modes de mise en oeuvre de l'invention

### Exemple 9 - Crème anti-rides

Une crème de jour anti-rides comportant le composé de formule (IIa), sous forme d'une émulsion huile (Phase A) dans eau (Phase B), comporte les ingrédients listés dans le tableau 1 ci-après.

**Tableau 1 - Constituants de la crème anti-rides et leurs proportions**

| Constituants | Proportions |
|---|---|
| Phase A | |
| Cetearyl alcohol et Cetearyl glucoside (Montanov® 68) | 5 % (p/v) |
| Huile de Jojoba | 5 % (p/v) |
| Huile de vaseline | 5 % (p/v) |
| Isopropyl palmitate | 7 % (p/v) |

| Phase B | |
|---|---|
| Glycérine | 5 % (p/v) |
| Allantoïde | 0,1 % (p/v) |
| Polyacrylamide et C13-14 Isoparaffine et Laureth-7 (Speigel® 305) | 0,3 % (p/v) |
| Rétinol | 0,05 % (p/v) |
| Phénoxyéthanol | 0,8 % (p/v) |
| Parfum | 0,1 % (p/v) |
| Composé (IIa) | 100 ppm |
| Eau déminéralisée | qsp 100 % |

### Exemple 10 - Lait pour le visage et le corps

Un lait pour le visage et le corps comportant le composé de formule (IIb), sous forme d'une émulsion huile (Phase A) dans eau (Phase B), comporte les ingrédients listés dans le tableau 2 ci-après.

**Tableau 2 - Constituants du lait et leurs proportions**

| Constituants | Proportions |
|---|---|
| Phase A | |
| Huile | 5 % (p/v) |
| Acide stéarique | 3 % (p/v) |
| Stéarate de glycéryle | 0,5 % (p/v) |
| Triéthanolamine | 0,5 % (p/v) |
| Alcool cétylique | 0,2 % (p/v) |

| Phase B | |
|---|---|
| Glycérine | 5 % (p/v) |
| Caféine | 0,5 % (p/v) |
| Carbomer | 0,2 % (p/v) |
| Méthyl paraben | 0,2 % (p/v) |
| Parfum | 0,05 % (p/v) |
| Composé (IIb) | 20 ppm |
| Eau déminéralisée | qsp 100 % |

### Exemple 11 - Crème hydratante

Une crème hydratante comportant le composé de formule (IIf), sous forme d'une émulsion huile (Phase A) dans eau (Phase B), comporte les ingrédients listés dans le tableau 3 ci-après.

**Tableau 3 - Constituants de la crème hydratante et leurs proportions**

| Constituants | Proportions |
|---|---|
| Phase A | |
| PEG-30 Dipolyhydroxystéarate Isononanoate (Arlacel® P135) | 2 % (p/v) |
| Isostéaryl Neopentanoate | 3 % (p/v) |
| Hydrogenated Polyisobutene | 3 % (p/v) |
| Octyldodecyl Stéarate | 9 % (p/v) |
| Ethylhexyl Palmitate | 3 % (p/v) |

| Phase B | |
|---|---|
| Sorbeth-30 | 5 % (p/v) |
| Magnesium Sulfate | 0,7 % (p/v) |
| Phénoxyéthanol et Methylparaben et Ethylparaben et Propylparaben (Rokonsal® MEP) | 0,5 % (p/v) |
| Parfum | 0,1 % (p/v) |
| Composé (IIf) | 10 ppm |
| Eau déminéralisée | qsp 100 % |

### EXEMPLES 12-16 - Etude de l'effet de composés selon des modes de mise en oeuvre de l'invention sur l'expression de gènes de cellules cutanées

Dans ces exemples, l'expression des ARN messagers a été mesurée par la technique de RT-PCR (transcription inverse suivie de réaction de polymérisation en chaîne) quantitative, après incubation in vitro des différents types cellulaires en absence ou en présence des composés testés.

Cette étude a été réalisée sur Fibroblastes dermiques humains normaux (NHDF) ou sur Kératinocytes épidermiques humains normaux (NHEK), selon les gènes testés. Les molécules ont été testées à 10⁻⁵ M dans une solution aqueuse à 0,1 % de diméthylsulfoxyde (DMSO).

Les cellules ont été mises en culture à la concentration de 10 000 cellules par puits de plaques 96 puits, en présence de milieu de culture standard en fonction du type cellulaire.

Les composés ont ensuite été ajoutés à la concentration étudiée, pendant 24 h.

Le surnageant a été éliminé et les cellules ont été reprises dans un tampon spécifique pour l'extraction des ARN messagers (ARNm). Les ARNm ont été purifiés et réverse-transcrits en présence d'une réverse transcriptase commerciale.

Les ADN complémentaires (ADNc) obtenus ont été utilisés dans une expérience de PCR quantitative en temps réel, au moyen d'amorces adéquates. Le taux d'expression des ARNm a été normalisé avec 5 gènes de référence.

### Exemple 12 - Effet de composés sur l'expression génique de MMP-9 (Matrice-Métalloprotéase de type 9)

Les composés de formule (IIa), (IIc), (IId), (IIe), (IIf) ont été testés.

L'acide caféique a été utilisé comme référence.

Les cellules utilisées pour cet exemple sont des kératinocytes humains normaux (NHEK).

La mise en contact avec les composés testés a été effectuée pendant 24 heures.

Les résultats obtenus, en termes de diminution de l'expression de MMP-9 (codée par le gène de numéro d'accession Genbank : NG_011468.1) par rapport au niveau basal (absence de composé) rapporté à 100 %, sont montrés sur le tableau 4 ci-après.

**Tableau 4 - Effet des composés sur l'expression de MMP-9**

| Composés testés | Diminution de l'expression de MMP-9 par rapport au niveau basal rapporté à 100 % |
|---|---|
| Acide caféique | Pas de diminution |
| (IIa) | - 67 % |
| (IIc) | - 56 % |
| (IId) | - 47 % |
| (IIe) | - 81 % |
| (IIf) | - 63 % |

Pour tous les composés conformes à l'invention testés, on observe une diminution significative du niveau d'expression de la protéine MMP-9. Une telle diminution induit un ralentissement de la dégradation des protéines de structure du derme.

### Exemple 13 - Effet de composés sur l'expression du gène codant la sirtuine-5

Les composés de formule (IIa), (IIb), (IIe), (IIf) ont été testés.

L'acide caféique a été utilisé comme référence.

Les cellules utilisées pour cet exemple sont des fibroblastes humains normaux (NHDF).

La mise en contact avec les composés testés a été effectuée pendant 24 heures.

Les résultats obtenus, en termes d'expression de la sirtuine-5 (codée par le gène numéro d'accession Genbank: NC_000006.11) par rapport au niveau basal (absence de composé) rapporté à 100 %, sont montrés sur le tableau 5 ci-après.

**Tableau 5 - Effet des composés sur l'expression de la sirtuine-5**

| Composés testés | Expression de la sirtuine-5 par rapport au niveau basal rapporté à 100 % |
|---|---|
| Acide caféique | 100 % |
| (IIa) | 153 % |
| (IIb) | 125 % |
| (IIe) | 146 % |
| (IIf) | 140 % |

Pour tous les composés conformes à l'invention testés, on observe une augmentation significative du niveau d'expression de la sirtuine-5. Une telle augmentation induit une augmentation de la survie cellulaire, par retard.de l'apoptose.

### Exemple 14 - Effet de composés sur l'expression du gène codant la protéine Klotho (KL)

Le composé de formule (IIb) a été testé.

Les cellules utilisées pour cet exemple sont des fibroblastes humains normaux (NHDF).

La mise en contact avec les composés testés a été effectuée pendant 24 heures.

Le résultat obtenu, en termes d'expression de la protéine Klotho (codée par le gène de numéro d'accession Genbank : NG_011485.1) par rapport au niveau basal (absence de composé) rapporté à 100 %, est égal à + 144 %. Une augmentation aussi significative de l'expression de cette protéine entraîne une amélioration de la survie de ces cellules de la peau.

### Exemple 15 - Effet de composés sur l'expression de gènes impliqués dans la différenciation et l'adhésion cellulaire

Le composé de formule (IIa) a été testé.

Les cellules utilisées pour cet exemple sont des kératinocytes humains normaux (NHEK).

La mise en contact avec les composés testés a été effectuée pendant 24 heures.

Les résultats obtenus, en termes d'expression de différents gènes par rapport au niveau basal (absence de composé) rapporté à 100 %, sont montrés sur le tableau 6 ci-après. Dans ce tableau, après le nom de chaque gène, est indiqué entre parenthèse le numéro d'accession Genbank.

**Tableau 6 - Effet du composé (IIa) sur l'expression de gènes impliqués dans la différenciation et l'adhésion cellulaire**

| Gènes testés | Expression génique par rapport au niveau basal rapporté à 100 % |
|---|---|
| EPPK1 (Epiplakine 1) (NC_000008.11) | 177 % |
| EVPL (Envoplakine) (NC_000017.10) | 166 % |
| TGM1 (Transglutaminase 1) (NG_007150.1) | 183 % |
| SPINK5 (Serine peptidase Kazal type 5) (NG_009633.1) | 173 % |
| AQP3 (Aquaporine 3) (G_007476.1) | 228 % |
| CRNN (Cornuline) (NC_000001.10) | 261 % |
| KRT1 (Kératine 1) (NG_008364.1) | 142 % |

On observe une augmentation significative du niveau d'expression de tous les gènes testés,. Une telle augmentation favorise notamment la formation et l'étanchéité de la fonction barrière cutanée.

### Exemple 16 - Effet de composés sur l'expression de gènes codant des protéines de la matrice extracellulaire

Le composé de formule (IIa) a été testé.

Les cellules utilisées pour cet exemple sont des fibroblastes humains normaux (NHDF).

La mise en contact avec les composés testés a été effectuée pendant 24 heures.

Les résultats obtenus, en termes d'expression de différents gènes par rapport au niveau basal (absence de composé) rapporté à 100 %, sont montrés sur le tableau 7 ci-après. Dans ce tableau, après le nom de chaque gène, est indiqué entre parenthèse le numéro d'accession Genbank.

**Tableau 7 - Effet du composé (IIa) sur l'expression de gènes codant des protéines de la matrice extracellulaire**

| Gènes testés | Expression génique par rapport au niveau basal rapporté à 100 % |
|---|---|
| COL1A11 (Collagène 1) (NG_007400.1) | 133 % |
| COL3A1 (Collagène 3) (NG_007404.1) | 125 % |
| COL5A1 (Collagène 5) (NG_008030.1) | 144 % |
| ELN (Elastine) (NG_009261.1) | 136 % |
| LAMA5 (Laminine alpha 5) (NC_000020.10) | 141 % |
| HAS3 (Hyaluronan synthase 3) (NC_000016.9) | 144 % |
| SPARC (Osteonectine) (NC_000005.9) | 118 % |

On observe une augmentation significative du niveau d'expression de tous les gènes testés. Une telle augmentation favorise notamment la néosynthèse de protéines de la matrice extracellulaire.

### Exemple 17 - Effet de composés selon l'invention et de composés comparatifs sur l'expression du Collagène 1

Dans cet exemple, l'expression du Collagène 1 a été mesurée au niveau protéique par la technique d'immunofluorescence, après incubation *in vitro* des cellules en absence ou en présence des composés testés, à une concentration de 10 ppm dans une solution aqueuse à 0,1 % de DMSO.

Ont été testés le composé conforme à l'invention de formule (IIa) et les composés comparatifs Comp.1 et Comp.2, de formules respectives (IV) et (V).

Le TGFβ a été utilisé comme témoin positif, à la concentration de 10 ng/ml dans une solution aqueuse. Un contrôle non traité a également été réalisé.

Cette étude a été réalisée sur Fibroblastes dermiques humains normaux (NHDF).

Les cellules ont été mises en culture à la concentration de 4000 cellules par puits de plaques 96 puits, en présence de milieu de culture standard.

Les composés ont ensuite été ajoutés à la concentration étudiée, identique pour tous, pendant 24h.

Le surnageant a été éliminé et les cellules ont été préparées pour le marquage fluorescent. Les cellules ont été fixées en présence de formaldéhyde 3,7 %, puis perméabilisées dans un tampon contenant 1% de Triton®, enfin les cellules ont été rincées et incubées en présence d'un anticorps monoclonal anti-Collagène1 (Abcam) pendant 1h30. Après lavage, les cellules ont été incubées en présence de l'anticorps secondaire (Alexa GAM488, Invitrogen) durant 1 h. Après lavage les cellules ont été incubées en présence de DAPI pendant 10 min, puis rincées une nouvelle fois.

La fluorescence a ensuite été quantifiée à l'aide d'un microscope automatisé (ArrayScan®, Cellomics®).

Les résultats obtenus, en termes d'expression du Collagène 1 par rapport au niveau basal (contrôle) rapporté à 100 %, sont montrés sur la figure 1.

On observe le composé conforme à l'invention (IIa) stimule de manière importante la production de collagène 1 par les cellules, et autant que le témoin positif (TGFβ). Au contraire, les composés comparatifs Comp.1 et Comp.2, bien que de structure proche du composé (IIa), ne stimulent pas, ou extrèmement peu (en tenant compte de l'écart-type) cette production de collagène 1.

La description ci-avant illustre clairement que par ses différentes caractéristiques et leurs avantages, la présente invention atteint les objectifs qu'elle s'était fixés. En particulier, elle fournit des composés dont l'utilisation, formulés dans une composition cosmétique destinée à une application par voie topique, infère sur les fonctions des cellules cutanées pour augmenter leur action anti-vieillissement de la peau, de sorte à prévenir, et même à réparer, les symptômes du vieillissement cutané.

### REFERENCES BIBLIOGRAPHIQUES

Bradshaw AD, Reed MJ, Sage EH. (2002), SPARC-null mice exhibit accelerated cutaneous wound closure, J Histochem Cytochem., 50(1):1-10
Chavanas S, Bodemer C, Rochat A, Hamel-Teillac D, Ali M, Irvine AD, Bonafé JL, Wilkinson J, Taïeb A, Barrandon Y, Harper JI, de Prost Y, Hovnanian A. (2000), Mutations in SPINK5, encoding a serine protease inhibitor, cause Netherton syndrome, Nat Genet., 25(2):141-2
Contzler R, Favre B, Huber M, Hohl D. (2005), Cornulin, a new member of the "fused gene" family, is expressed during epidermal differentiation, J Invest Dermatol., 124(5):990-7
Eckert RL, Sturniolo MT, Broome AM, Ruse M, Rorke EA. (2005), Transglutaminase function in epidermis, J Invest Dermatol., 124(3):481-92
Jang SI, Kalinin A, Takahashi K, Marekov LN, Steinert PM (2005), Characterization of human epiplakin: RNAi-mediated epiplakin depletion leads to the disruption of keratin and vimentin IF networks, J Cell Sci. 118(Pt 4):781-93
Hara-Chikuma M, Verkman AS (2005), Aquaporin-3 functions as a glycerol transporter in mammalian skin, Biol Cell., 97(7):479-86
Karashima T, Watt FM. (2002), Interaction of periplakin and envoplakin with intermediate filaments, J Cell Sci., 115(Pt 24):5027-37
Kuo HJ, Maslen CL, Keene DR, Glanville RW. (1996), Type VI collagen anchors endothelial basement membranes by interacting with type IV collagen, J Biol Chem., 272(42):26522-9
Kuro-o M. (2009), Klotho and aging. Biochim Biophys Acta, 1790(10):1049-58
Manuskiatti W, Maibach HI. (1996), Hyaluronic acid and skin: wound healing and aging, Int J Dermatol., 35(8):539-44
Schlicker C, Gertz M, Papatheodorou P, Kachholz B, Becker CF, Steegborn C (2008), Substrates and régulation mechanisms for the human mitochondrial sirtuins Sirt3 and Sirt5, J Mol Biol., 382(3):790-801
Seltzer JL, Eisen AZ, Bauer EA, Morris NP, Glanville RW, Burgeson RE. (1989), Cleavage of type VII collagen by interstitial collagenase and type IV collagenase (gelatinase) derived from human skin, J Biol Chem., 264(7):3822-6
Senshu T, Kan S, Ogawa H, Manabe M, Asaga H. (1996), Preferential deimination of keratin K1 and filaggrin during the terminal differentiation of human epidermis, Biochem Biophys Res Commun., 225(3):712-9
Schneider H, Mühle C, Pacho F. (2006), Biological function of laminin-5 and pathogenic impact of its deficiency, Eur J Cell Biol., 86(11-12):701-17
Tzaphlidou M. (2004), The role of collagen and elastin in aged skin: an image processing approach, Micron., 35(3):173-7
Uitto J. (1979), Biochemistry of the elastic fibers in normal connective tissues and its alterations in diseases, J Invest Dermatol., 72(1):1-10

## Revendications

1. Utilisation pour stimuler les fonctions cellulaires anti-vieillissement de la peau d'un composé de formule générale (I) : 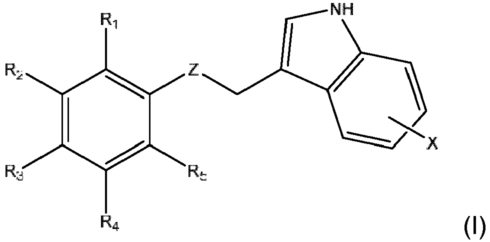 dans laquelle :
R₁, R₂, R₃, R₄, R₅, identiques ou différents, représentent chacun un atome d'hydrogène, un atome d'halogène, un groupement hydroxyle ou un radical -OR' où R' représente un radical hydrocarboné linéaire ou ramifié, saturé ou insaturé, en C1-C16, au moins un groupement parmi R₁, R₂, R₃, R₄ et R₅ ne représentant pas un atome d'hydrogène ou un atome d'halogène,
X représente un atome d'halogène, un groupement hydroxyle, un groupement nitro, un radical hydrocarboné linéaire ou ramifié, saturé ou insaturé, éventuellement substitué, en C1-C14, ou un radical -OR" où R" représente un radical hydrocarboné linéaire ou ramifié, saturé ou insaturé, éventuellement substitué, en C1-C14,
Z représente une liaison covalente ou un bras espaceur,
ou un de ses sels,
dans une composition cosmétique.

2. Utilisation selon la revendication 1, **caractérisée en ce que** ledit composé répond à la formule (II) : 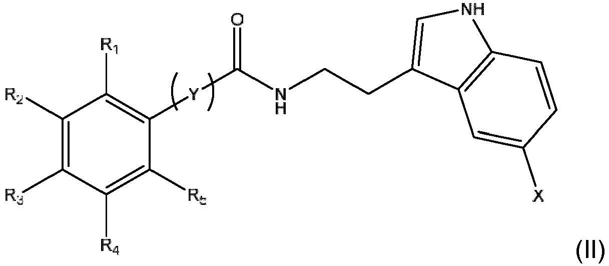 dans laquelle Y représente une liaison covalente ou un radical hydrocarboné linéaire ou ramifié, saturé ou insaturé, en C1-C16.

3. Utilisation selon la revendication 2, **caractérisée en ce que**, dans la formule (II), au moins un parmi R₃, R₄ et R₅ représente un groupement hydroxyle, X représente un groupement hydroxyle ou un groupement méthoxy, et Y représente une liaison covalente ou un radical alcényle en C2-C4.

4. Utilisation selon la revendication 3, **caractérisée en ce que** ledit composé répond à la formule (IIa) :

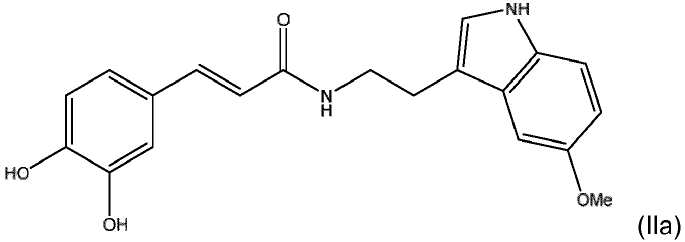

5. Utilisation selon la revendication 3, **caractérisée en ce que** ledit composé répond à la formule (IIb) :

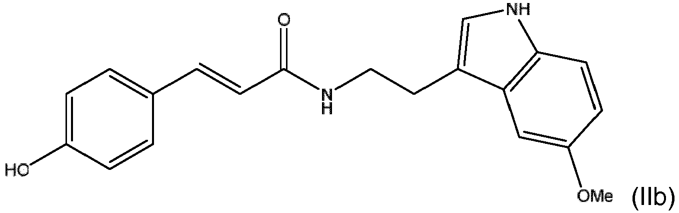

6. Utilisation selon l'une quelconque des revendications 1 à 5, **caractérisée en ce que** ledit composé est présent dans ladite composition cosmétique à une concentration comprise entre 0,0000001 et 10 % en poids du poids total de la composition, de préférence à une concentration comprise entre 0,00001 et 1 % en poids du poids total de la composition.

7. Utilisation selon l'une quelconque des revendications 1 à 6, **caractérisée en ce que** ladite composition cosmétique est destinée à un usage topique.

8. Utilisation selon l'une quelconque des revendications 1 à 7, **caractérisée en ce que** ladite composition cosmétique se présente sous forme de crème, de pommade, de lait, d'huile, d'onguent, de lotion, de poudre, de solution, de gel, de suspension, de savon, de tampon imbibé, ou encore de shampooing.

9. Utilisation selon l'une quelconque des revendications 1 à 8, **caractérisée en ce que** ladite composition cosmétique comporte au moins un ingrédient actif supplémentaire.

## Patentansprüche

1. Verwendung einer Verbindung mit der allgemeinen Formel (I) zur Stimulierung der zellulären Alterungshemmungsfunktionen der Haut: 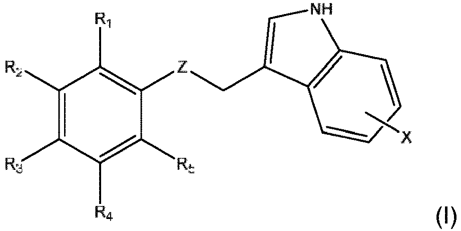 wobei:
R₁, R₂, R₃, R₄, R₅, die identisch oder unterschiedlich sind, jeweils für ein Wasserstoffatom, ein Halogenatom, eine Hydroxylgruppe oder ein -OR'-Radikal stehen, wobei R' für ein lineares oder verzweigtes, gesättigtes oder ungesättigtes Kohlenwasserstoffradikal mit C1-C16 steht, wobei wenigstens eine Gruppe aus R₁, R₂, R₃, R₄ und R₅ nicht für ein Wasserstoffatom oder ein Halogenatom steht,
X für ein Halogenatom, eine Hydroxylgruppe, eine Nitro-Gruppe, ein lineares oder verzweigtes, gesättigtes oder ungesättigtes, gegebenenfalls substituiertes Kohlenwasserstoffradikal mit C1-C14 oder ein -OR"-Radikal steht, wobei R" für ein lineares oder verzweigtes, gesättigtes oder ungesättigtes, gegebenenfalls substituiertes Kohlenwasserstoffradikal mit C1-C14 steht,
Z für eine kovalente Bindung oder einen Linker steht,
oder eines ihrer Salze,
in einer kosmetischen Zusammensetzung.

2. Verwendung nach Anspruch 1, **dadurch gekennzeichnet, dass** die Verbindung der Formel (II) entspricht: 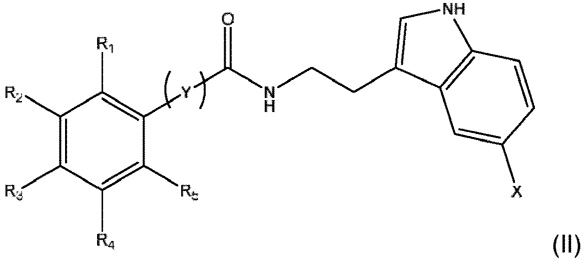 wobei Y für eine kovalente Bindung oder für ein lineares oder verzweigtes, gesättigtes oder ungesättigtes Kohlenwasserstoffradikal mit C1-C16 steht.

3. Verwendung nach Anspruch 2, **dadurch gekennzeichnet, dass** in der Formel (II) wenigstens eines aus R₃, R₄ und R₅ für eine Hydroxylgruppe steht, X für eine Hydroxylgruppe oder eine Methoxygruppe steht und Y für eine kovalente Bindung oder ein Alkenylradikal mit C2-C4 steht.

4. Verwendung nach Anspruch 3, **dadurch gekennzeichnet, dass** die Verbindung der Formel (IIa) entspricht:

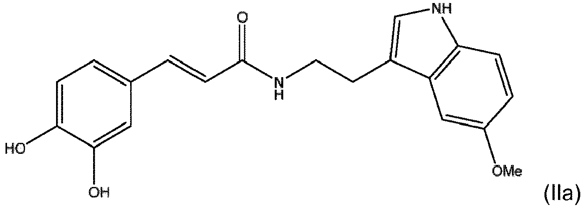

5. Verwendung nach Anspruch 3, **dadurch gekennzeichnet, dass** die Verbindung der Formel (IIb) entspricht:

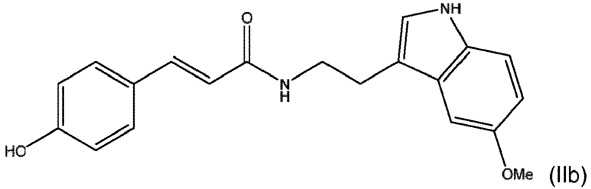

6. Verwendung nach einem der Ansprüche 1 bis 5, **dadurch gekennzeichnet, dass** die Verbindung in der kosmetischen Zusammensetzung in einer Konzentration zwischen 0,0000001 und 10 Gew.-% bezogen auf das Gesamtgewicht der Zusammensetzung, vorzugsweise in einer Konzentration zwischen 0,00001 und 1 Gew.-% bezogen auf das Gesamtgewicht der Zusammensetzung vorhanden ist.

7. Verwendung nach einem der Ansprüche 1 bis 6, **dadurch gekennzeichnet, dass** die kosmetische Zusammensetzung für eine topische Anwendung bestimmt ist.

8. Verwendung nach einem der Ansprüche 1 bis 7, **dadurch gekennzeichnet, dass** die kosmetische Zusammensetzung in Form einer Creme, einer Pomade, einer Milch, eines Öls, einer Salbe, einer Lotion, eines Puders, einer Lösung, eines Gels, einer Suspension, einer Seife, eines getränkten Bausches oder eines Shampoos vorliegt.

9. Verwendung nach einem der Ansprüche 1 bis 8, **dadurch gekennzeichnet, dass** die kosmetische Zusammensetzung wenigstens einen zusätzlichen aktiven Inhaltsstoff enthält.

## Claims

1. The use for stimulating the anti-aging cell functions of the skin, of a compound of general formula (I): 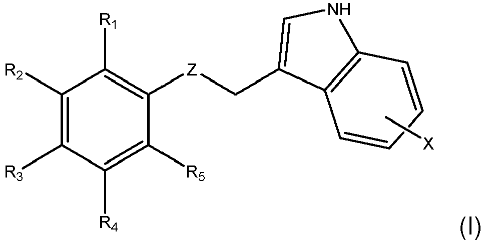 in which:
R₁, R₂, R₃, R₄, R₅, which may be identical or different, each represent a hydrogen atom, a halogen atom, a hydroxyl group or a radical -OR' in which R' represents a linear or branched, saturated or unsaturated C1-C16 hydrocarbon-based radical, at least one group from among R₁, R₂, R₃, R₄ and R₅ not representing a hydrogen atom or a halogen atom,
X represents a halogen atom, a hydroxyl group, a nitro group, a linear or branched, saturated or unsaturated, optionally substituted, C1-C14 hydrocarbon-based radical, or a radical -OR" in which R" represents a linear or branched, saturated or unsaturated, optionally substituted, C1-C14 hydrocarbon-based radical,
Z represents a covalent bond or a spacer arm,
or a salt thereof,
in a cosmetic composition.

2. The use as claimed in claim 1, **characterized in that** said compound corresponds to formula (II): 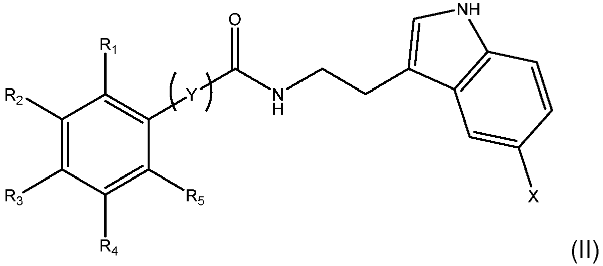 in which Y represents a covalent bond or a linear or branched, saturated or unsaturated, C1-C16 hydrocarbon-based radical.

3. The use as claimed in claim 2, **characterized in that**, in formula (II), at least one from among R₃, R₄ and R₅ represents a hydroxyl group, X represents a hydroxyl group or a methoxy group, and Y represents a covalent bond or a C2-C4 alkenyl radical.

4. The use as claimed in claim 3, **characterized in that** said compound corresponds to formula (IIa):

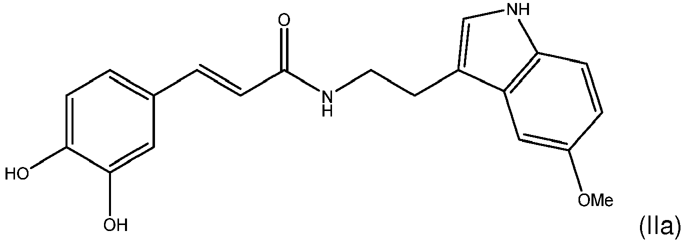

5. The use as claimed in claim 3, **characterized in that** said compound corresponds to formula (IIb):

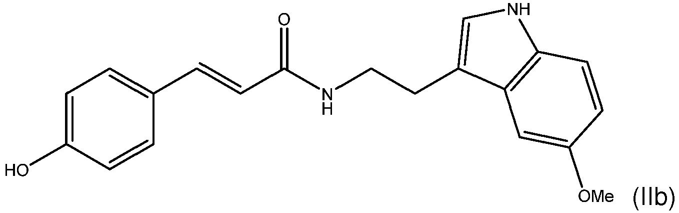

6. The use as claimed in any one of claims 1 to 5, **characterized in that** said compound is present in said cosmetic composition at a concentration of between 0.0000001% and 10% by weight relative to the total weight of the composition, and preferably at a concentration of between 0.00001 % and 1 % by weight relative to the total weight of the composition.

7. The use as claimed in any one of claims 1 to 6, **characterized in that** said cosmetic composition is intended for topical use.

8. The use as claimed in any one of claims 1 to 7, **characterized in that** said cosmetic composition is in the form of a cream, a pomade, a milk, an oil, an ointment, a lotion, a powder, a solution, a gel, a suspension, a soap, an impregnated pad, or a shampoo.

9. The use as claimed in any one of claims 1 to 8, **characterized in that** said cosmetic composition comprises at least one additional active ingredient.
